# EUROPEAN PATENT APPLICATION

(11) **EP 2 679 197 A1**
(43) Date of publication of application: **01.01.2014**
(21) Application number: 13174409.6
(22) Date of filing: 28.06.2013
(51) Int. Cl.: A61F 2/07, A61F 2/00

(54) **Sealing mechanism for expandable vascular graft**

(30) Priority: 30.06.2012 US 201261666858 P; 24.06.2013 US 201313925814
(71) Applicant: Cordis Corporation, Bridgewater, NJ 08807 (US)
(72) Inventor: Schroeder, Valeska, Menlo Park, CA California 94025 (US)
(74) Representative: Carpmaels & Ransford LLP

(57) **Abstract**

A stent-graft device for repairing aneurysms and or providing a fluid conduit in the body is disclosed. In particular the stent-graft device has primary and secondary sealing mechanisms that provide flow diversion at the sealing neck of an aneurysm repair device.

## Description

### CROSS REFERENCE TO RELATED APPLICATION

This application claims the benefit of U.S. Provisional Application, Serial Number 61/666,858 filed June 30, 2012, which is incorporated by reference herein.

### BACKGROUND OF THE INVENTION

### 1. Field of the Invention

The present invention relates to stent-graft devices, and more particularly, to a secondary sealing mechanism and device that provides flow diversion at the sealing neck of an aneurysm repair device.

### 2. Discussion of the Related Art

An aneurysm is an abnormal dilation of a layer or layers of an arterial wall, usually caused by a systemic collagen synthetic or structural defect. An abdominal aortic aneurysm is an aneurysm in the abdominal portion of the aorta, usually located in or near one or both of the two iliac arteries or near the renal arteries. The aneurysm often arises in the infrarenal portion of the diseased aorta, for example, below the kidneys. A thoracic aortic aneurysm is an aneurysm in the thoracic portion of the aorta. When left untreated, the aneurysm may rupture, usually causing rapid fatal hemorrhaging.

Aneurysms may be classified or typed by their position as well as by the number of aneurysms in a cluster. Typically, abdominal aortic aneurysms may be classified into five types. A Type I aneurysm is a single dilation located between the renal arteries and the iliac arteries. Typically, in a Type I aneurysm, the aorta is healthy between the renal arteries and the aneurysm and between the aneurysm and the iliac arteries.

A Type II A aneurysm is a single dilation located between the renal arteries and the iliac arteries. In a Type II A aneurysm, the aorta is healthy between the renal arteries and the aneurysm, but not healthy between the aneurysm and the iliac arteries. In other words, the dilation extends to the aortic bifurcation. A Type II B aneurysm comprises three dilations. One dilation is located between the renal arteries and the iliac arteries. Like a Type II A aneurysm, the aorta is healthy between the aneurysm and the renal arteries, but not healthy between the aneurysm and the iliac arteries. The other two dilations are located in the iliac arteries between the aortic bifurcation and the bifurcations between the external iliacs and the internal iliacs. The iliac arteries are healthy between the iliac bifurcation and the aneurysms. A Type II C aneurysm also comprises three dilations. However, in a Type II C aneurysm, the dilations in the iliac arteries extend to the iliac bifurcation.

A Type III aneurysm is a single dilation located between the renal arteries and the iliac arteries. In a Type III aneurysm, the aorta is not healthy between the renal arteries and the aneurysm. In other words, the dilation extends to the renal arteries.

A ruptured abdominal aortic aneurysm is presently the thirteenth leading cause of death in the United States. The routine management of abdominal aortic aneurysms has been surgical bypass, with the placement of a graft in the involved or dilated segment. Although resection with a synthetic graft via a transperitoneal or retroperitoneal procedure has been the standard treatment, it is associated with significant risk. For example, complications include perioperative myocardial ischemia, renal failure, erectile impotence, intestinal ischemia, infection, lower limb ischemia, spinal cord injury with paralysis, aorta-enteric fistula, and death. Surgical treatment of abdominal aortic aneurysms is associated with an overall mortality rate of five percent in asymptomatic patients, sixteen to nineteen percent in symptomatic patients, and is as high as fifty percent in patients with ruptured abdominal aortic aneurysms.

Disadvantages associated with conventional surgery, in addition to the high mortality rate, include an extended recovery period associated with the large surgical incision and the opening of the abdominal cavity, difficulties in suturing the graft to the aorta, the loss of the existing thrombosis to support and reinforce the graft, the unsuitability of the surgery for many patients having abdominal aortic aneurysms, and the problems associated with performing the surgery on an emergency basis after the aneurysm has ruptured. Further, the typical recovery period is from one to two weeks in the hospital and a convalescence period, at home, ranging from two to three months or more, if complications ensue. Since many patients having abdominal aortic aneurysms have other chronic illnesses, such as heart, lung, liver and/or kidney disease, coupled with the fact that many of these patients are older, they are less than ideal candidates for surgery.

The occurrence of aneurysms is not confined to the abdominal region. While abdominal aortic aneurysms are generally the most common, aneurysms in other regions of the aorta or one of its branches are possible. For example, aneurysms may occur in the thoracic aorta. As is the case with abdominal aortic aneurysms, the widely accepted approach to treating an aneurysm in the thoracic aorta is surgical repair, involving replacing the aneurysmal segment with a prosthetic device. This surgery, as described above, is a major undertaking, with associated high risks and with significant mortality and morbidity.

Over the past five years, there has been a great deal of research directed at developing less invasive, endovascular, i.e., catheter directed, techniques for the treatment of aneurysms, specifically abdominal aortic aneurysms. This has been facilitated by the development of vascular stents, which can and have been used in conjunction with standard or thin-wall graft material in order to create a stent-graft or endograft. The potential advantages of less invasive treatments have included reduced surgical morbidity and mortality along with shorter hospital and intensive care unit stays.

Stent-grafts or endoprostheses are now Food and Drug Administration (FDA) approved and commercially available. Their delivery procedure typically involves advanced angiographic techniques performed through vascular accesses gained via surgical cut down of a remote artery, which may include the common femoral or brachial arteries. Over a guidewire, the appropriate size introducer will be placed. The catheter and guidewire are passed through the aneurysm. Through the introducer, the stent-graft will be advanced to the appropriate position. Typical deployment of the stent-graft device requires withdrawal of an outer sheath while maintaining the position of the stent-graft with an inner-stabilizing device. Most stent-grafts are self-expanding; however, an additional angioplasty procedure, e.g., balloon angioplasty, may be required to secure the position of the stent-graft. Following the placement of the stent-graft, standard angiographic views may be obtained.

Due to the large diameter of the above-described devices, typically greater than twenty French (3F=1 mm), arteriotomy closure typically requires open surgical repair. Some procedures may require additional surgical techniques, such as hypogastric artery embolization, vessel ligation, or surgical bypass in order to adequately treat the aneurysm or to maintain blood flow to both lower extremities. Likewise, some procedures will require additional advanced catheter directed techniques, such as angioplasty, stent placement and embolization, in order to successfully exclude the aneurysm and efficiently manage leaks.

While the above-described endoprostheses represent a significant improvement over conventional surgical techniques, there is a need to improve the endoprostheses, their method of use and their applicability to varied biological conditions. Accordingly, in order to provide a safe and effective alternate means for treating aneurysms, including abdominal aortic aneurysms and thoracic aortic aneurysms, a number of difficulties associated with currently known endoprostheses and their delivery systems must be overcome. One concern with the use of endoprostheses is the prevention of endo-leaks and the disruption of the normal fluid dynamics of the vasculature. Devices using any technology should preferably be simple to position and reposition as necessary, should preferably provide an acute, fluid tight seal, and should preferably be anchored to prevent migration without interfering with normal blood flow in both the aneurysmal vessel as well as branching vessels. In addition, devices using the technology should preferably be able to be anchored, sealed, and maintained in bifurcated vessels, tortuous vessels, highly angulated vessels, partially diseased vessels, calcified vessels, odd shaped vessels, short vessels, and long vessels. In order to accomplish this, the endoprostheses should preferably be highly durable, extendable and reconfigurable while maintaining acute and long-term fluid tight seals and anchoring positions.

The endoprostheses should also preferably be able to be delivered percutaneously utilizing catheters, guidewires and other devices which substantially eliminate the need for open surgical intervention. Accordingly, the diameter of the endoprostheses in the catheter is an important factor. This is especially true for aneurysms in the larger vessels, such as the thoracic aorta. In addition, the endoprostheses should preferably be percutaneously delivered and deployed such that surgical cut down is unnecessary.

The repair device should also be able to maintain fluid tight seals, especially in devices comprising a number of independent interlocking or overlapping components.

### SUMMARY OF THE INVENTION

The present invention overcomes the disadvantages associated with currently utilized aneurismal repair devices.

In accordance with one aspect, the present invention is directed to an anchoring and sealing component having a substantially tubular stent structure. A mesh structure is coaxially disposed over and affixed to at least a portion of the stent structure. A graft material is disposed over and affixed to at least a portion of the stent structure and a portion of the mesh structure. The graft material and the mesh structure are oriented such that the distal portion of the mesh structure extends distally past the graft material.

### BRIEF DESCRIPTION OF THE DRAWINGS

The foregoing and other features and advantages of the invention will be apparent from the following, more particular description of preferred embodiments of the invention, as illustrated in the accompanying drawings.
Figure 1 is a diagrammatic representation of the exemplary anchoring and sealing prosthesis in accordance with one embodiment of the present invention.
Figure 2 is a diagrammatic representation of an exemplary endovascular graft in accordance with the present invention.
Figure 3 is a diagrammatic representation of a first exemplary four-point suture knot in accordance with the present invention.
Figure 4 is a diagrammatic representation of a second exemplary four-point suture knot in accordance with the present invention.
Figure 5 is a diagrammatic representation of a first exemplary four-point suture knot having modified ends in accordance with the present invention.
Figure 6 is a diagrammatic representation of a second exemplary four-point suture knot having modified ends in accordance with the present invention.
Figure 7 is a diagrammatic representation of exemplary suture knot holding structures on a stent segment in accordance with the present invention.
Figure 8 is a diagrammatic representation of a section of graft material attached to a stent segment with modified suture knots in accordance with the present invention.
Figure 9 is a diagrammatic representation of exemplary anchoring and sealing prosthesis deployed in an Aortic Aneurysm in accordance with one embodiment of the present invention.
Figure 10A is a magnified view of a portion of the diagrammatic representation illustrated in Figure 9.
Figure 10B is a magnified view of a portion of the diagrammatic representation illustrated in Figure 9.
Figure 11 is a diagrammatic representation of a mesh according to one embodiment of the present invention.
Figure 12 is a diagrammatic representation of a mesh according to one embodiment of the present invention.

### DETAILED DESCRIPTION OF THE PREFERRED EMBODIMENTS

Aneurysm repair devices make take on a wide variety of configurations. Aneurysm repair devices may comprise one element configurations or multiple element or modular element configurations. The secondary sealing mechanisms of the present invention may be utilized with many types of aneurysm repair devices that rely on a primary seal of fabric mesh against the vessel wall. While Abdominal Aortic Aneurysm (AAA) repair devices are used as specific examples, it is contemplated that this unique sealing mechanism can be used with many other types of devices at various other locations.

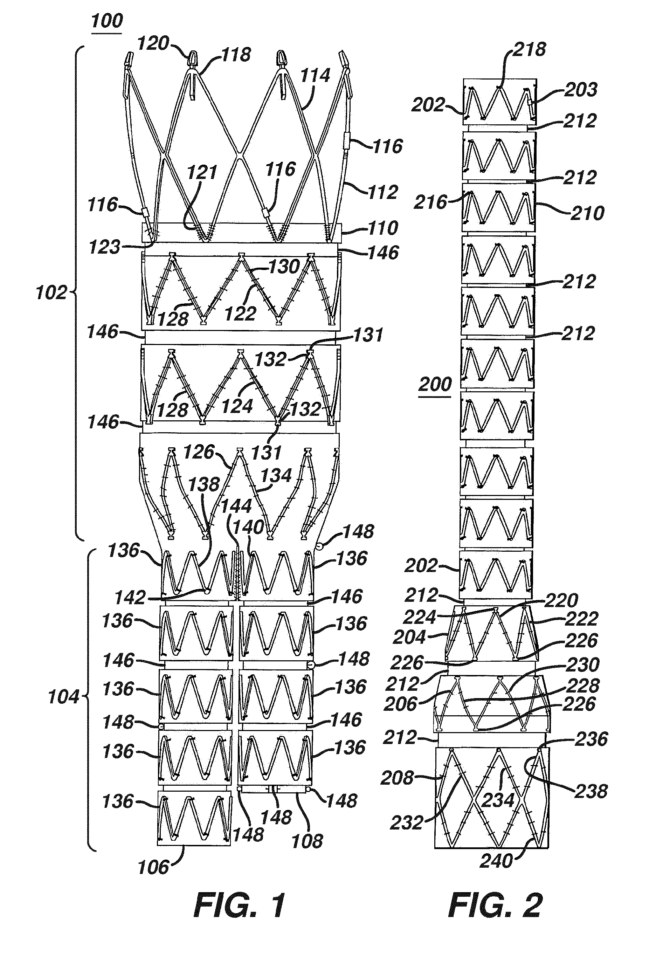

Referring to Figure 1, there is illustrated an exemplary anchoring and sealing component 100 of a modular aneurysm repair device. The anchoring and sealing component 100 comprises a trunk section 102 and a bifurcated section 104, which includes two legs 106 and 108. Graft material 110, described in detail below, and may be affixed to at least a portion of the trunk section 102 and to both of the legs 106 and 108. The combination of the graft material and the underlying scaffold structures creates a blood carrying conduit for insertion into a vessel. The graft material 110 may be attached to the underlying scaffold structures via any suitable means. In the exemplary embodiment set forth herein, the graft material 110 is attached to portions of the underlying scaffold structures by sutures. As is explained in detail subsequently, the types of sutures utilized as well as the type of stitches may be varied depending on their location and function. The sutures may comprise any suitable biocompatible material that is preferably highly durable and wear resistant.

The use of the terms proximal and distal are in this application are used to indicate a relative position or direction. As applied to AAA devices, proximal indicates a position or direction closer to the heart, while distal indicates a position or direction away from the heart.

The underlying scaffold structures of the trunk section 102 comprise a number of substantially tubular stent structures, which may be formed from any number of suitable materials. The upper or proximal end of the trunk section 102 comprises a first stent segment 112 having a diamond shaped configuration formed from a plurality of struts 114. Marker bands 116 formed from a highly radiopaque material such as tantalum may be positioned at various locations on the struts 114 for imaging purposes during device implantation. In other words, the markers 116 may help the physician to visualize the device under radio fluoroscopy. At the upper apex 118 of each diamond cell is an eyelet and barb structure 120. The eyelet portion is utilized in conjunction with a delivery system while the barb portion is utilized to affix the anchoring and sealing component 100 in the vessel into which it is placed. As may be readily seen from Figure 1, the upper portion of the first stent segment 112 is not covered with graft material 110. This portion is not covered so that it does not interfere with or otherwise impede blood flow to or from cross or branch arteries, for example, the renal arteries. The lower portion of the first stent segment 112; however, is covered by graft material 110. Sutures 121 are utilized to secure the graft material to the lower or distal apexes 123 of the first stent segment 112.

The lower or distal portion of the trunk section 102 comprises three individual stent segments 122, 124 and 126. Stent segments 122 and 124 are identical in design, with each comprising a single row of struts 128 arranged in a substantially zigzag configuration. Sutures 130 are utilized to attach the graft material 110 to each of the stent segments 122 and 124. In addition, each stent segment 122 and 124 comprises a suture locking mechanism 131 on at least one upper and lower apex 132. These suture locking mechanisms 131 allow for special suture knots to secure the graft material 110 to the stent segments 122 and 124. It has been determined that these locations are subject to wear due to high biological forces and thus additional securing mechanisms are utilized to prevent separation of the graft material 110. Stent segment 126 is identical to stent segments 122 and 124 with one exception. Specifically, the struts 134 forming this third stent segment 126 are tapered inward in the circumferential direction thereby causing the diameter of the lower portion of the trunk section 102 to decrease where it connects to the bifurcated section 104. As with the other two stent segments 122 and 124, stent segment 126 also comprises suture locking mechanisms 131 on at least one upper and lower apex 132.

As described above, the bifurcated section 104 includes two legs 106 and 108. As may be readily seen from Figure 1, leg 106 is longer than leg 108. This configuration eases deliverability. Each leg 106 and 108 is otherwise identical, and comprises a plurality of individual, substantially tubular stent segments 136. Each stent segment 136 comprises a single row of struts 138 arranged in a substantially zigzag configuration. Sutures 140 are utilized to secure the graft material 110 to the stent segments 136. These sutures 140, unlike sutures 130 and 121 are only utilized to secure the graft material 110 proximate the apexes 142 of the stent segments 136 rather than along the entire length of a strut. Each leg 106 and 108 is free to move independently of each other; however, proximate the junction with the trunk section 102, the graft material 110 of each leg 106 and 108 is stitched together with sutures 144. This is done to prevent tearing of the graft material 110 if and when the legs 106 and 108 move.

It is important to note that the graft material 110 covering the anchoring and sealing component 100 comprises crimped sections 146 between the various underlying scaffold elements. These crimped sections increase the flexibility of the entire device. A detailed description of the graft materials and how the crimps are formed is given subsequently.

In use, the anchoring and sealing component 100 is percutaneously positioned in a blood vessel with one or more aneurysms. It is anchored in healthy tissue above the aneurysm and serves as the first conduit to bypass the diseased section of the artery. Additional stent-graft components or endovascular grafts attach to the legs 106 and 108 to extend the bypass to healthy tissue beyond the aneurysm. The system is designed as a modular system so that as many extensions as necessary may be utilized. Essentially, the additional or modular components overlap and form an interference fit. This particular exemplary embodiment having two legs is specifically designed for branching into two vessels, for example, from the abdominal aortic artery to the iliac arteries. However, other similar modular components may be utilized in any other suitable artery.

To aid in the correct positioning of the anchoring and sealing component 100, additional markers 148 are affixed to the device in various locations. The additional markers 148 may be formed out of any suitable, highly radiopaque material such as tantalum. The additional markers 148 may be attached to either or both of the underlying stent structures and the graft material by any suitable means, including stitches and glue. In the exemplary embodiment, the additional markers 148 are attached to the graft material.

Referring now to Figure 2, there is illustrated an exemplary embodiment of an endovascular graft 200 of the aneurysm repair device. The exemplary endovascular graft 200 comprise one or more first stent segments 202, a second stent segment 204, a third stent segment 206 and a fourth stent segment 208. Graft material 210 is attached to the stent segments 202, 204, 206 and 208 to form a substantially tubular conduit. As in the above described design, crimped sections 212 are formed in the graft material 210 between the stent segments to increase flexibility. In a typical use scenario, the fourth stent segment 208 would be anchored in healthy tissue below the aneurysm and a number of the uppermost first stent segments 202 would overlap with one of the legs 106 and 108 of the anchoring and sealing component 100 thereby establishing a fluid channel through the diseased section of the artery. The degree of overlap may vary. Obviously the greater the degree of overlap, the less likely the chance of separation. In addition, as is explained in detail subsequently, the stent-graft suture locks of the present invention have more chances to engage with a higher degree of overlap. In this exemplary embodiment, a second endovascular graft would be connected to the second leg. As stated above, additional endovascular grafts may be connected together if longer conduits are required to bypass the diseased tissue.

The one or more first stent segments 202 each comprises a single row of struts 214 arranged in a substantially zigzag configuration. Sutures 216 are utilized to secure the graft material 210 to the stent segments 202. These sutures 216 are only utilized to secure the graft material 210 proximate the apexes 218 of the first stent segments 202 rather than along the entire length of a strut forming the segment 202. The diameter of the one or more first stent segments 202 with the graft material 210 attached thereto is substantially equal to that of either of the legs 106 and 108 such that a tight interference fit may be achieved when the components are attached. In the exemplary embodiment, the endovascular graft 200 fits inside of the legs 106 and 108; however, in alternate exemplary embodiments wherein the endovascular graft 200 fits over or outside of the legs 106 and 108. As described in detail below, knots on the sutures of the cranial or proximal end of the endovascular graft 200 help anchor the endovascular graft within the legs 106 and 108.

The upper or distal most first stent segment 202 comprises a marker band 203 for positioning the device. Once again, the marker band 203 may comprise any suitable, highly radiopaque material such as tantalum. It is important to note that while a number of different markers are illustrated, additional markers that are not shown are positioned at various locations around each of the components so that the physician may easily visualize the device under radio fluoroscopy.

The second segment 204 comprises a single row of struts 220 arranged in a substantially zigzag configuration. The diameter of the stent segment 204 is slightly larger than the diameter of the stent segment 202. The increase in diameter may be achieved through the use of longer struts. Sutures 222 are utilized to secure the graft material 210 to the stent segment 204. These sutures 222 are only utilized to secure the graft material 210 proximate the apexes 224 of the second stent segment 204 rather than along the entire length of a strut forming the segment 204. In addition, each stent segment 204 comprises a suture locking mechanism 226 on at least one upper and lower apex 224. These suture locking mechanisms 226 allow for special suture knots to secure the graft material 210 to the stent segment 204. It has been determined that these locations are subject to wear due to high biological forces and thus additional securing mechanism are utilized to prevent separation of the graft material 210.

Third stent segment 206 is identical to stent segment 204 with one exception. Specifically, the struts 228 forming this third stent segment 206 are tapered outward in the circumferential direction thereby causing the diameter of the lower portion of the endovascular graft 200 to increase where it anchors in the vessel. Sutures 230 are utilized to secure the graft material 210 to the third sent segment 206. As with the second stent segment 204, third stent segment 206 also comprises suture locking mechanisms 226 on at least one upper and lower apex 224. The diameter of the third stent segment 206 is substantially equal to the diameter of the second stent segment 204 on one end and substantially equal to the diameter of the fourth stent segment 208 on the other end.

The fourth stent segment 208 has a diamond shaped configuration formed from a plurality of struts 232. Sutures 234 are utilized to secure the graft material 210 to the fourth stent segment 208. Suture locking mechanisms 236 are utilized on one or more apexes 238 only on the end of the fourth stent segment 208 proximate the third stent segment 206. The fourth stent segment 208 also comprises at least one marker band 240 attached to a strut for imaging the device. As described above, the marker band 240 may comprise any suitable, highly radiopaque material such as tantalum.

All of the stent segments described herein are substantially tubular elements that may be formed utilizing any number of techniques and any number of materials. In the preferred exemplary embodiment, all of the stent segments are formed from a nickel-titanium alloy (Nitinol), shape set laser cut tubing.

Nitinol is utilized in a wide variety of applications, including medical device applications as described herein. Nitinol or Ni-Ti alloys are widely utilized in the fabrication or construction of medical devices for a number of reasons, including its biomechanical compatibility, its biocompatibility, its fatigue resistance, its kink resistance, its uniform plastic deformation, its magnetic resonance imaging compatibility, its constant and gentle outward pressure, its dynamic interference, its thermal deployment capability, its elastic deployment capability, its hysteresis characteristics and because it is modestly radiopaque.

Nitinol, as described above, exhibits shape memory and/or super elastic characteristics. Shape memory characteristics may be simplistically described as follows. A metallic structure, for example a Nitinol tube that is in an Austenite phase may be cooled to a temperature such that it is in the Martensite phase. Once in the Martensite, the Nitinol tube may be deformed into a particular configuration or shape by the application of stress. As long as the Nitinol tube is maintained in the Martensite phase, the Nitinol tube will remain in its deformed shape. If the Nitinol tube is heated to a temperature sufficient to cause the Nitinol tube to reach the Austenite phase, the Nitinol tube will return to its original or programmed shape. The original shape is programmed to be a particular shape by well known techniques. Super elastic characteristics may be simplistically described as follows. A metallic structure, for example, a Nitinol tube that is in an Austenite phase may be deformed to a particular shape or configuration by the application of mechanical energy. The application of mechanical energy causes a stress induced Martensite phase transformation. In other words, the mechanical energy causes the Nitinol tube to transform from the Austenite phase to the Martensite phase. By utilizing the appropriate measuring instruments, one can determine that the stress from the mechanical energy causes a temperature drop in the Nitinol tube. Once the mechanical energy or stress is released, the Nitinol tube undergoes another mechanical phase transformation back to the Austenite phase and thus its original or programmed shape. As described above, the original shape is programmed by well known techniques. The Martensite and Austenite phases are common phases in many metals.

Medical devices constructed from Nitinol are typically utilized in both the Martensite phase and/or the Austenite phase. The Martensite phase is the low temperature phase. A material in the Martensite phase is typically very soft and malleable. These properties make it easier to shape or configure the Nitinol into complicated or complex structures. The Austenite phase is the high temperature phase. A material in the Austenite phase is generally much stronger than the material in the Martensite phase. Typically, many medical devices are cooled to the Martensite phase for manipulation and loading into delivery systems, as described above with respect to stents and then when the device is deployed at body temperature, they return to the Austenite phase.

All of the stent segments are preferably self-expandable and formed from a shape memory alloy. Such an alloy may be deformed from an original, heat-stable configuration to a second, heat-unstable configuration. The application of a desired temperature causes the alloy to revert to an original heat-stable configuration. A particularly preferred shape memory alloy for this application is binary nickel titanium alloy comprising about 55.8 percent Ni by weight, commercially available under the trade designation NITINOL. This NiTi alloy undergoes a phase transformation at physiological temperatures. A stent made of this material is deformable when chilled. Thus, at low temperatures, for example, below twenty degrees centigrade, the stent is compressed so that it can be delivered to the desired location. The stent may be kept at low temperatures by circulating chilled saline solutions. The stent expands when the chilled saline is removed and it is exposed to higher temperatures within the patient's body, generally around thirty-seven degrees centigrade.

In preferred embodiments, each stent is fabricated from a single piece of alloy tubing. The tubing is laser cut, shape-set by placing the tubing on a mandrel, and heat-set to its desired expanded shape and size.

In preferred embodiments, the shape setting is performed in stages at five hundred degrees centigrade. That is, the stents are placed on sequentially larger mandrels and briefly heated to five hundred degrees centigrade. To minimize grain growth, the total time of exposure to a temperature of five hundred degrees centigrade is limited to five minutes. The stents are given their final shape set for four minutes at five hundred fifty degrees centigrade, and then aged to a temperature of four hundred seventy degrees centigrade to import the proper martensite to austenite transformation temperature, then blasted, as described in detail subsequently, before electro polishing. This heat treatment process provides for a stent that has a martensite to austenite transformation which occurs over a relatively narrow temperature range; for example, around fifteen degrees centigrade.

To improve the mechanical integrity of the stent, the rough edges left by the laser cutting are removed by combination of mechanical grit blasting and electro polishing. The grit blasting is performed to remove the brittle recast layer left by the laser cutting process. This layer is not readily removable by the electro polishing process, and if left intact, could lead to a brittle fracture of the stent struts. A solution of seventy percent methanol and thirty percent nitric acid at a temperature of minus forty degrees centigrade or less has been shown to work effectively as an electro polishing solution. Electrical parameters of the electro polishing are selected to remove approximately 0.00127 cm of material from the surfaces of the struts. The clean, electro polished surface is the final desired surface for attachment to the graft materials. This surface has been found to import good corrosion resistance, fatigue resistance, and wear resistance.

The graft material utilized to cover all of the stent segments may be made from any number of suitable biocompatible materials, including woven, knitted, sutured, extruded, or cast materials comprising polyester, polytetrafluoroethylene, silicones, urethanes, and ultra light weight polyethylene, such as that commercially available under the trade designation SPECTRA™. The materials may be porous or nonporous. Exemplary materials include a woven polyester fabric made from DACRON™ or other suitable PET-type polymers.

In one exemplary embodiment, the fabric for the graft material is a forty denier (denier is defined in grams of nine thousand meters of a filament or yarn), twenty-seven filament polyester yarn, having about seventy to one-hundred end yarns per cm per face and thirty-two to forty-six pick yarns per cm face. At this weave density, the graft material is relatively impermeable to blood flow through the wall, but is relatively thin, ranging between 0.08 and 0.12 mm in wall thickness.

Prior to attachment of the graft component to the stent segments, crimps are formed between the stent positions by placing the graft material on a shaped mandrel and thermally forming indentations in the surface. In the exemplary embodiment illustrated in Figures 1 and 2, the crimps 146 and 212 respectively, are about two mm long and 0.5 mm deep. With these dimensions, the endovascular graft can bend and flex while maintaining an open lumen. Also, prior to attachment of the graft material to the stent segments, the graft material is cut in a shape to conform to the shapes of the stent segments.

As stated above, the graft material is attached to each of the stent segments. The graft material may be attached to the stent segments in any number of suitable ways. In the exemplary embodiment, the graft material is attached to the stent segments by sutures.

The method of suturing stents in place is important for minimizing the relative motion or rubbing between the stent struts and the graft material. Because of the pulsatile motion of the vasculature and therefore the entire device, it is possible for relative motion to occur, particularly in areas where the device or component thereof is in a bend, or if there are residual folds in the graft material, due to being constrained by the aorta or iliac arteries.

Depending on the stent segments location, different types of sutures may be utilized. In the exemplary embodiment illustrated in Figure 1, in the lower portion of the first stent segment 112, the graft material 110 with sutures 121 using a blanket type stitch. For stent segments 122, 124 and 126, the graft material is attached with sutures 130 using a blanket type stitch. For the stents segments 136, the graft material is attached with sutures 140 using point type stitches. In the exemplary embodiment illustrated in Figure 2, the graft material 210 is attached to first stent segments by sutures 216 using point type stitches. For stent segment 204, the graft material 210 is attached using sutures 222 using blanket type stitches. For stent segment 206, the graft material 210 is attached using sutures 230 using blanket type stitches. For stent element 208, the graft material 210 is attached using sutures 234 using blanket type stitches.

In accordance with the present invention, the suture knots utilized to fasten the graft material to the underlying stent structures may be modified to enhance the overall performance of the aneurysm repair device. Essentially, the modified suture knots may be utilized to create tailored profiles that increase the ability of one component to adhere the other components and/or vessels to prevent component separation or migration. For example, a certain percentage of the point type stitches of the endovascular graft 200 may be modified so that it will be better secured within the legs 106 and 108.

The modified suture knots of the present invention as described herein may be utilized with all stent-graft devices that have overlapping or modular components to prevent separation thereof as well as those components that are prone to migration within a vessel. This, as is explained in detail subsequently, is accomplished by placing or positioning the modified suture knots in locations on the stent-graft component that overlaps with another component or that comes into contact with the vessel wall.

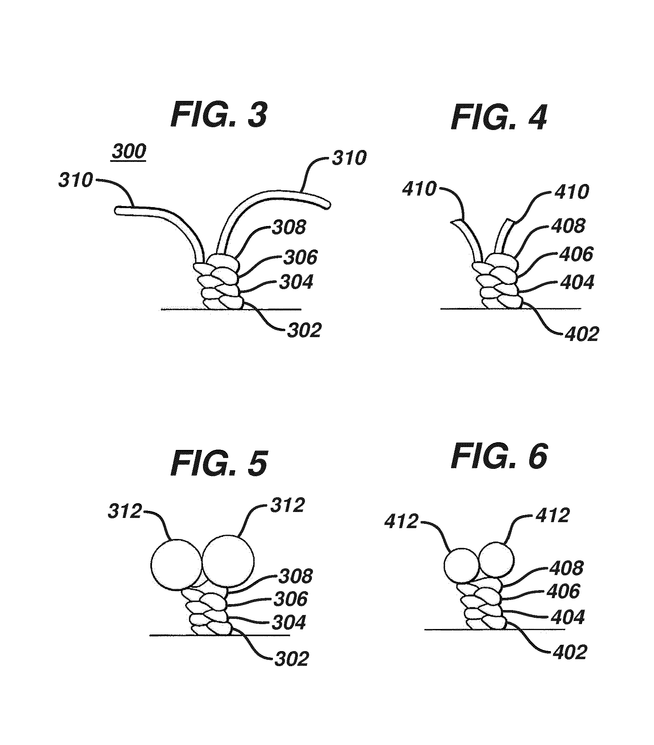

As described above, sutures are utilized on stent-graft structures for the sole purpose of holding the graft material to the underlying stent structure. The knots to tie off the ends of the suture stitch, typically four-throw point type stitches, are commonly known as a Surgeon's knot and is illustrated in Figure 3. The Surgeon's knot 300 illustrated is a four-throw point stitch. Four-throw point knots are beneficial in that the four throws 302, 304, 306 and 308 are less likely to unravel. The Surgeon's knot illustrated in Figure 3 has long ends 310, whereas the Surgeon's knot 400 illustrated in Figure 4 has four throws 402, 404, 406 and 408, but short ends 410. It is important to note that suture knots with less throws may be utilized, for example, identical knots with two throws. In addition, it is important to note that different types of knots may be utilized as long as its ends are free to be modified as described below.

The suture knots may be modified in any number of ways in accordance with the present invention. In a preferred exemplary embodiment, the ends of the suture knots are cauterized be heating the free ends to create retention elements or structures which are spherical as illustrated in Figures 5 and 6. As may be readily understood and referring to Figures 5 and 6, the suture knots 300 with longer free ends 310 have larger spherical retention elements or structures 312 than the spherical retention elements or structures 412 of the suture knots 400 with shorter free ends 410. These melted spherical retention elements or structures provide for a variety of knot profiles that depend on the number of throws and the lengths of the free ends prior to melting. It is important to note that the retention structures or elements may comprise any suitable shape and/or configuration. Essentially, they are formed by melting the ends of the suture knots and thus can be molded into a variety of shapes having a larger diameter than the diameter of the suture. Without any molding, the heated ends simply melt into a spherical or ball like shape. In other embodiments, additional elements may be affixed to the ends of the sutures to form the retention elements or as referred to in the present invention, stent-graft suture locks.

In operation, these modified knots 300 and 400 may be positioned around a specific element that is inserted into another element such that the underlying stent structure of the one component interacts with the knots in a manner that substantially prevents relative movement there between. In other words, devices having modular components and that are subject to high biological forces for extended periods of time are constrained from relative movement because the knots of one component are essentially connected to at least a portion of the underlying structure of the other component. For example, if the sutures 216 of the first stent elements 202 of the endovascular graft 200 are knotted in accordance with the present invention, then when the endovascular graft 200 is inserted into one of the legs 106 or 108 of the anchoring and sealing component 100, the modified knots will interlock with at least one element of stent segments 136.

Alternately, if the modified knots are being utilized to anchor the component to the vessel, then the sutures 140 on the stent segments 136 of the legs 106 and 108 as well as the sutures 216 on the stent segments 202 of the endovascular graft 200 that do not overlap with the legs 106 and 108 may have modified knots that function to grab onto the inner wall of the vessel into which the components are positioned. In this manner the modified knots substantially prevent movement of the devices relative to the vessel.

The modified knots may interact with corresponding knots on associated components, with the underlying stent structures of associated components, with any other suitable structure on the associated component or the vessel into which it is implanted.

What is described above is a number of exemplary embodiments of the invention. However, it is important to note that the retention elements or locks can assume any profile and/or shape and may be located or positioned at any suitable position both inside and outside of the graft structures. For example, arrays of retention elements or locks may be positioned around the graft elements. The retention elements or locks may be formed from suture knots that do not also function to secure the graft material to the underlying stent structures. Essentially, any configuration may be utilized. In addition, a briefly described above, the degree of overlap of components may also determine the location, size, number and shape of the retention elements or locks.

It is also important to note that the locks may be utilized on other devices, for example, grafts formed without underlying stent structures.

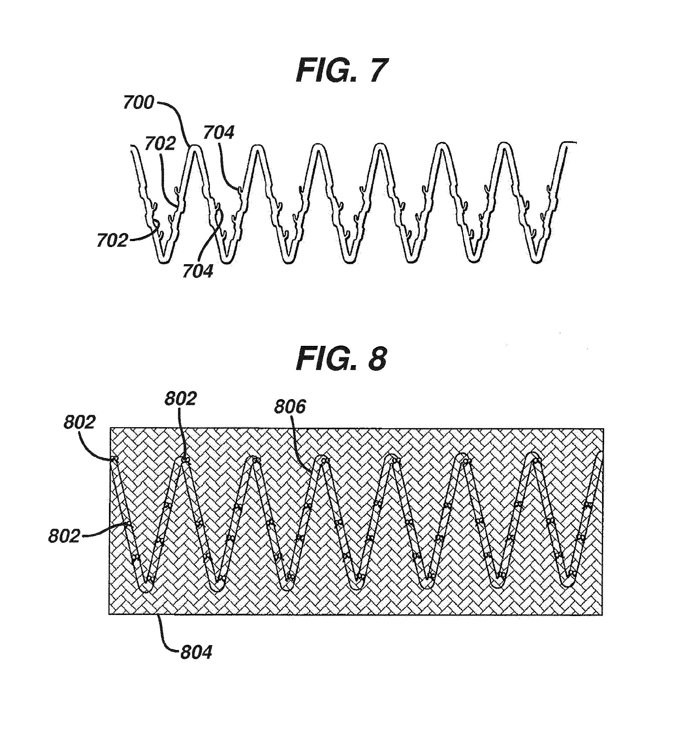

Referring to Figure 7 and Figure 8, there is illustrated a stent segment 700 having indents 702 and/or protrusions/hooks 704 which may be utilized to lock onto the retention elements formed from the suture knots of the present invention as well the modified suture knots 802 securing the graft material 804 to the stent segment 806. As illustrated, any of the modified suture knots 802 can lock onto any of the indents 702 and/or protrusions/hooks 704. With this configuration of knots and locking points, the chances for them connecting are much greater. In other words, by having a number of modified suture knots 802 positioned in various locations around the stent segment 806 and then having a number of indents 702 and/or protrusions/hooks 704 positioned around the stent segment, the greater chance of making a locking connection. These indents 702 and/or protrusions/hooks 704 may comprise any suitable configuration for grabbing and holding the retention elements from another component.

It is important to note the retention elements of the present invention, in all of its forms, may be utilized on or in conjunction with any stent, stent-graft, and/or graft and not just for ones for repairing abdominal aortic aneurysms or aneurysms in general.

The strength of anchoring or locking may be demonstrated by a review of the following data. An experiment was done to determine the force (pull-out force) required to separate two sets of components, one set with the suture locks of the present invention and one set without the suture locks. The experiment simply involved measuring the maximum force required to separate the components, or more particularly, to dislodge an endovascular graft from the leg of the anchoring and sealing component. In the experiment, water at body temperature was circulated through the components to simulate normal body conditions as closely as possible. In addition, an overlap of two stent segments was utilized. Other degrees of overlap may be utilized without departing from the scope and spirit of the invention. In the experiment with the components without the suture locks, the test was repeated three times with the following results: 17.7 N maximum force required to separate the two components, 8.9 N maximum force to separate the two components and 10.9 N maximum force to separate the two components, resulting in an average maximum pull-out force of 12.5 N. In the experiment with the components with the suture locks, the test was repeated two times with the following results: 21.1 N maximum force to separate the two components and 20.5 N maximum force to separate the two components, resulting in an average pull-out force of 20.8 N. It is clear from the data that the suture locks of the present invention results in an over sixty-five (65) percent increase in the pull-out force.

Finally, it is important to note that the modified suture knots of the present invention perform another function which provides a major benefit in the construction of the device. The modified suture knots prevent the ends of the knot from coming undone and having the entire knot unravel.

One potential problem with aneurysm repair devices, particularly endovascular anchoring and sealing devices, is their susceptibility to endo-leaks. Typically endovascular anchoring and sealing devices, particularly AAA devices, have a Nitinol or other shape memory structure that applies a chronic outward force to hold the fabric graft material against the vessel wall. In AAA devices, this is typically just below the renal arteries. As described above, AAA devices also typically have leg members, such as graft 200 that seal against the walls of the iliac arteries. Endo-leaks occur when fluid being excluded from the aneurysm, this case arterial blood, leaks between the end of the sealing member and the arterial wall, passing the sealing member, and back into the aneurysm. Endo-leaks can become critical issues with AAA devices, and may cause catastrophic failure resulting in open surgical repair, or even death of the patient.

To reduce the risk and/or potential severity of endo-leaks, one embodiment of the present invention includes a secondary flow diversion mechanism and seal, which is particularly helpful when the primary sealing mechanism is insufficient or damaged. The design of the frames or graft are not critical to the design of this secondary flow diversion mechanism and seal.

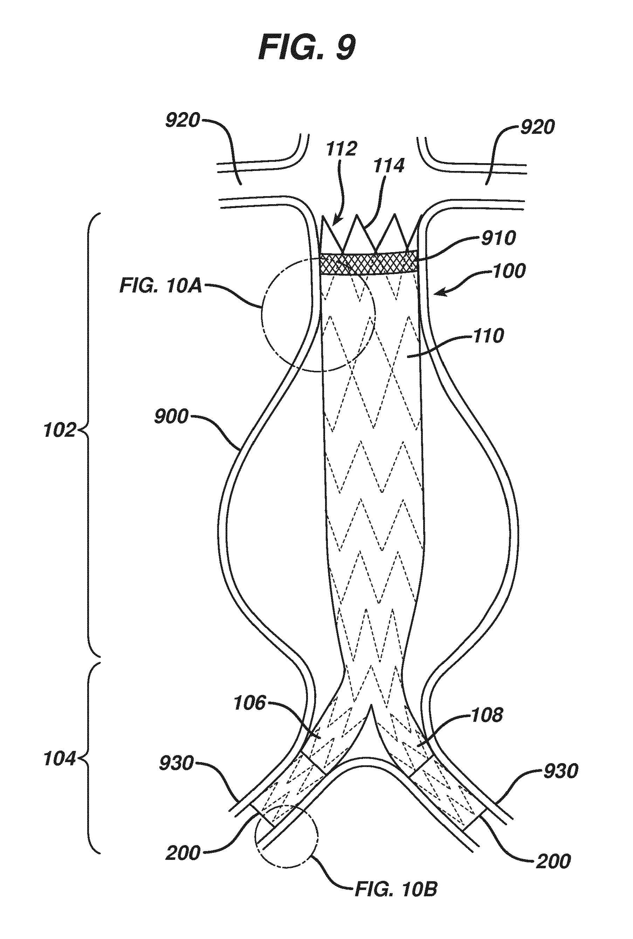

Referring to Figure 9, there is illustrated an exemplary anchoring and sealing component 100 comprising a trunk section102 and bifurcated section 104, which includes two legs 106 and 108. Graft material 110 is affixed to at least a portion of the trunk section 102 and to both leg sections 106 and 108. The combination of the graft material and the underlying scaffold structure creates a blood carrying conduit for insertion into a vessel. In this exemplary embodiment, the anchoring and sealing component is deployed in aorta 900, between the renal arteries 920 and iliac arties 930 to bypass and aortic aneurysm 940.

The underlying scaffold structure of the trunk section 102 comprises a number of substantially tubular stent structures, which may be formed from any number of suitable materials. The term "substantially" as used in this application is defined as having its ordinary meaning of "largely but now wholly that which is specified". The upper or proximal end of the trunk section 102 comprises a first stent segment 112 having a diamond shaped configuration formed from a plurality of struts 114. As may be readily seen from Figure 9, the upper portion of the first stent segment 112 is not covered with a graft material 110. This portion is not covered so that it does not interfere with or otherwise impede blood flow to or from cross or branch arteries, for example the renal arteries. The lower or distal portion of the first stent segment 112, however, is covered by graft material 110. As the stent segment 112 expands into the artery wall, the graft material 110 contacts the arterial wall, anchoring the device and providing the first or primary seal between the anchoring and sealing component 100 and the artery wall just below the renal arteries (suprarenal seal). This first seal assists in diverting the blood through the luminal interior and bypass the aneurysm. The anchoring and sealing component 100 may have a similar configuration at the distal end of the device, i.e. at the distal end of the legs 106, 108 or endovascular graft 200. See Figure 2. In the depicted embodiment this seal is between the iliac arteries and the appropriate stent segment 202, 204, 204, but particularly 208 (iliac seals). To provide a secondary seal, the anchoring and sealing component 100 further includes a mesh 910 coaxially disposed between the stent segment and graft material 110. In one preferred embodiment, the sealing mesh 910 is made from Nitinol wire mesh or machined Nitinol sheet 910. The machined Nitinol creates hole in the Nitinol sheet to make the sheet Nitinol porous.

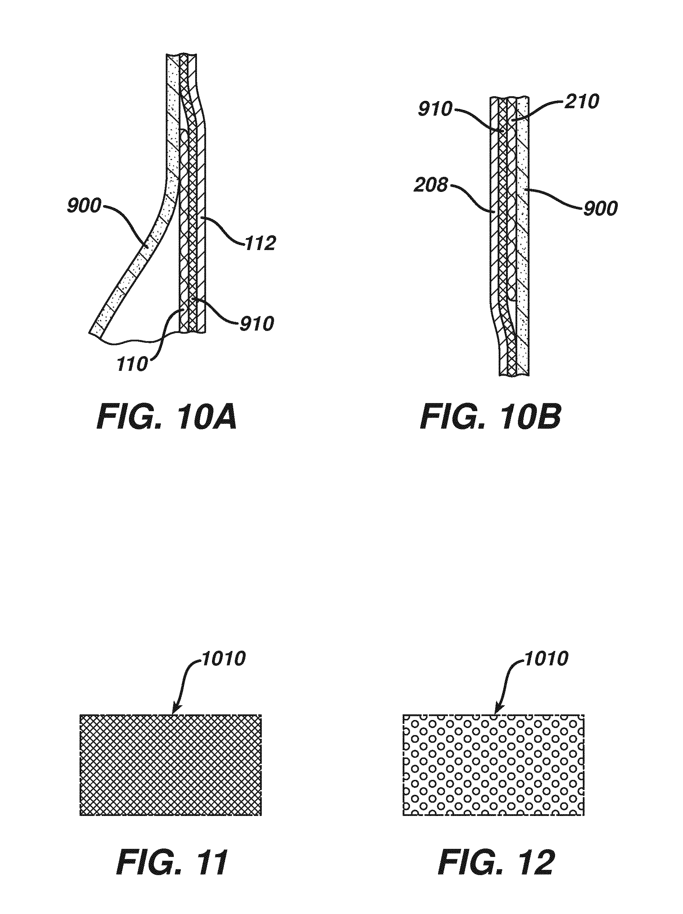

Figure 10 is a close up section view of a portion of anchoring and sealing component 100 illustrating the relationship between the stent segment 112, structural frame, the mesh 910 and fabric 110. As can be seen, the primary seal is located along the portion of the graft 110 that is immediately in contact with the vessel wall, aorta 900 in this instance. The Nitinol wire mesh or laser machined porous Nitinol 910 is attached to the stent segment 112 in the vicinity of the suprarenal seal. The Nitinol wire mesh may also be attached to the stent in the vicinity of the iliac seals on the distal ends of the legs 200. Attachment can be by any means known in the art, such as laser welding, stitching the mesh 910 to the frame 112, or adhesives. In addition, direct compressive forced between the frame 112, mesh 910 and vessel wall 900 may also hold the mesh 910 in place.

In the trunk section 102, the mesh 910 overlaps the fabric graft material 110 and extends in the proximal direction at the proximal end of the fabric graft 110 primary seal, and parallels the stent segment 112. The chronic outward force exerted by the expansion of the stent segment 112 naturally holds the portion of the mesh 910, extending proximally past the graft 110, against the vessel wall 900. This interface between the mesh 910, vessel wall 900, and stent segment 112 forms the secondary seal.

In the leg section 200, the mesh 910 overlaps the fabric graft material 210 and extends in the distal direction at the proximal end of the fabric graft 210 primary seal, and parallels the stent segment 208. The chronic outward force exerted by the expansion of the stent segment 208 naturally holds the portion of the mesh 910, extending distally past the graft 210, against the vessel wall 900. This interface between the mesh 910, vessel wall 900, and stent segment 208 forms the secondary seal.

The mesh 910 may be a mesh as illustrated in Figure 11, or alternatively a machined sheet as illustrated in Figure 12. In either case the mesh 910 is preferably made from a nickel titanium alloy, such as Nitinol. Nitinol provides goo biocompatibility and can be crimped down for delivery effectively. However other metals may be able to provide similar results.

The density of wire mesh 910 shown in Figure 11 may vary, as can the porosity of the machined Nitinol sheet illustrated in Figure 12. In either case, the open space (density) or porosity is ideally such that sufficient fluid can be diverted to relieve initial pressure from the primary seal. It is also important that the mesh 910 be sufficiently open to act as a scaffold for tissue growth. Tissue growth, some of which is a reaction caused by tissue injury that results from the implantation, will take place within the mesh 910, integrating the tissue mesh 910 into the vessel wall 900 and effectively anchoring and sealing the anchoring and sealing component 100 in place. Growth factors can be added to the mesh 910 surface to encourage tissue in-growth to create this additional flow diversion.

Immediately upon implantation of the anchoring and sealing component 100, the mesh 910 starts to divert some flow away from the primary seal and into the luminal passageway created by the anchoring and sealing component 100. This eases the pressure on the primary seal, allowing for a greater seal. Because the mesh 910 is porous, some fluid does pass the secondary seal and reaches the primary seal. With time, the tissue in-growth would allow for even greater or possible complete flow diversion.

Although shown and described is what is believed to be the most practical and preferred embodiments, it is apparent that departures from specific designs and methods described and shown will suggest themselves to those skilled in the art and may be used without departing from the spirit and scope of the invention. The present invention is not restricted to the particular constructions described and illustrated, but should be constructed to cohere with all modifications that may fall within the scope for the appended claims.

## Claims

1. A stent graft device for providing flow diversion through a body lumen, the body lumen having a luminal wall defining a luminal interior, the stent graft comprising:
a substantially tubular stent structure;
a substantially tubular, porous mesh structure coaxially disposed over and affixed to at least a portion of the stent structure; and
substantially tubular graft material disposed over and affixed to at least a portion of the stent structure and a portion of the mesh structure, the graft material and the mesh structure being oriented such that an end portion of the mesh structure extends past an end portion of the adjacent graft material, the stent structure and the graft material being configured to create a first seal between the stent graft device and the luminal wall of the body lumen, the substantially tubular stent structure and the mesh structure being configured to create a second seal between the stent graft device and the luminal wall.

2. The stent graft of claim 1 wherein the mesh structure is a wire mesh.

3. The stent graft of claim 1 wherein the mesh structure is a machined sheet.

4. The stent graft of claim 3 wherein the machined sheet is perforated.

5. The stent graft of claims 2 or 3 wherein the porosity of the mesh structure is sufficiently closed to divert fluid from the first seal and relieve pressure from the first seal, while being sufficiently open to act as a scaffold for tissue growth.

6. The stent graft of claims 2 or 3 wherein the mesh structure is made from Nitinol.

7. The stent graft of claim 1 wherein the mesh structure is affixed to the stent structure by laser welding.

8. The stent graft of claim 1 wherein the mesh structure is affixed to the stent structure by stitching.

9. The stent graft of claim 1 wherein the mesh structure is affixed to the stent structure by adhesives.

10. The stent graft of claim 1 wherein the mesh structure is affixed to the stent structure by the chronic outward pressure exerted by the stent against the mesh structure and luminal wall.

11. The stent graft of claim 1 wherein the end portion of the stent structure extends past the end portion of the mesh structure.

12. The stent graft of claim 1 further comprising growth factors added to the mesh structure.

13. The stent graft of claim 1 wherein the stent structure is made from Nitinol.

14. The stent graft of claim 1 wherein the graft material is made from a PET-type polymer.
